Europäisches Patentamt

European Patent Office

Office européen des brevets

(11) Publication number: **0 029 332**
**A1**

(12) **EUROPEAN PATENT APPLICATION**

(21) Application number: 80304018.7

(22) Date of filing: 11.11.80

(51) Int. Cl.³: **A 61 K 7/16**

(30) Priority: 15.11.79 JP 147011/79
15.11.79 JP 147012/79

(43) Date of publication of application: 27.05.81
Bulletin 81/21

(84) Designated Contracting States: DE FR GB

(71) Applicant: **DENTAL CHEMICAL CO., LIMITED,
3-1-1 Tsukiji Chuo-ku, Tokyo (JP)**

(72) Inventor: **Aoki, Hideki, 5-26, Yakuendai, Funabashi-shi
Chiba-Ken (JP)**

(74) Representative: **Stephens, Michael John, M.J. Stephens
& Co. Royal Building 11 St. Andrew's Cross, Plymouth
Devon PL1 2DS (GB)**

(54) **Dentifrice compositions.**

(57) The dentifrice composition contains synthetic hydroxy-apatite powder which is preferably neutral or weakly alkaline and may contain 0.1 to 20% by weight of NaCl and/or KCl and 0.003 to 3% by weight of $MgCl_2$. The dentifrice composition is very effective in eliminating plaque from teeth and, particularly when containing the above chlorides, has a fortifying and remineralizing effect on tooth surfaces.

EP 0 029 332 A1

- 1 -

## DENTIFRICE COMPOSITIONS

The invention relates to dentifrice compositions.

According to the present invention there is provided a dentifrice composition containing synthetic hydroxyapatite powder. The composition is preferably neutral or weakly alkaline, the preferred maximum pH value being substantially 9.

It has been found that the dentifrice composition of the present invention is effective in eliminating plaque (a colony of bacteria) from a tooth surface and also has some fortifying and remineralizing effect on the enamel coating of the tooth.

In general, a conventional dentifrice contains, as a tooth-cleaning and abrading agent, a mineral, such as colloidal silica, etc. While these abrading agents are used to remove contaminants on teeth, it is essential that they do not harm the teeth.

The dentifrice composition of the present invention contains a suitable amount of synthetic hydroxyapatite powder which can remove plaque as well as substances contaminating the teeth very effectively. We presume this is due to the fact that the synthetic hydroxyapatite has a hardness similar to that of the enamel portion of the tooth and can impart an appropriate abrading effect on the enamel portion during brushing of the teeth. We further presume that this is also due to the fact that the synthetic hydroxyapatite which, in preferred compositions has an average particle size of the hydroxyapatite powder of about $2\mu$ and a maximum particle size thereof $10\mu$ or less, has a large surface area and an excellent absorptivity.

In accordance with the present invention the dentifrice composition may further contain 0.1 to 20% by weight of NaCl and/or KCl and 0.003 to 3% by weight of $MgCl_2$, based on the weight of the composition.

0029332

- 2 -

It has been found that the presence of a mixture of NaCl and/or KCl with $MgCl_2$ in a dentifrice composition containing synthetic hydroxyapatite powder can effectively promote the elimination of plaque from teeth and enhance the fortification and remineralization of the surfaces of the teeth compared with compositions without these chlorides.

More specifically, the latter dentifrice composition of the present invention can deposit crystals of hydroxyapatite on the surfaces of the teeth more effectively than compositions without these chlorides. We presume this is due to the fact that the solubility of hydroxyapatite in water is increased in the presence of the chlorides specified above. More particularly, since hydroxyapatite is a salt which is only slightly soluble in water, the ion products of its ions ($Ca^{++}$), ($HPO_4^{--}$), etc., dissolved and ionised in water in the dentifrice, are small. This means that the ion products present for promoting coating of the surfaces of the teeth, i.e. depositing crystals of hydroxyapatite on the surfaces of the teeth, are small. If, however, NaCl and/or KCl, and $MgCl_2$ are added to the dentifrice containing hydroxyapatite, the solubilities of $Ca^{++}$ and $HPO_4^{--}$ are considerably increased thus, presumably, resulting in the observed enhancement of the coating effect on the teeth.

In cases where the amounts of NaCl and/or KCl, and $MgCl_2$ contained in the dentifrice composition of the present invention are smaller than those specified above, the enhancement of the coating effect, compared with that achieved by compositions of the invention without these chlorides, is insufficient to be of much advantage while, in compositions having amounts larger than those specified above, it becomes difficult to give a comfortable feeling in the use of the dentifrice composition.

- 3 -                                    0029332

The dentifrice composition of the present invention
may include various additives which are commonly employed
in dentifrices and may further include, if desired,citric
acid, lactic acid, acetic acid, pyrrolic acid, glutamine,
proline, serine, glycine, etc.

The invention will be further illustrated with
reference to the following examples. Examples 1 to 7
and 11 to 17 respectively show formulations of the
dentifrice compositions in accordance with the present
invention in terms of parts by weight and Examples 8 to 10,
18 and 19 are to substantiate effects of the compositions
of the present invention.

### Example 1.

#### Tooth Paste

| | |
|---|---|
| Hydroxyapatite Powder | 13.2 |
| Calcium phosphate | 25.0 |
| CMC sodium salt | 0.3 |
| Carrageenin | 1.2 |
| Glycerin | 10.0 |
| Sorbitol | 15.0 |
| Sodium lauryl sulphate | 2.0 |
| Flavour | 1.2 |
| Sodium saccharinate | 0.1 |
| Silicon Dioxide | 2.0 |
| Water | 30.0 |

### Example 2.

#### Tooth Paste

| | |
|---|---|
| Hydroxyapatite Powder | 7.2 |
| Calcium phosphate | 10.0 |
| Calcium pyrophosphate | 20.0 |
| CMC Sodium salt | 1.0 |
| Sodium alginate | 0.1 |
| Glycerin | 10.0 |
| Sorbitol | 10.0 |
| Sodium lauryl sulphate | 1.5 |
| Sodium lauryl sarcosinate | 0.5 |
| Flavour | 0.5 |
| Sodium saccharinate | 0.1 |
| Silicon dioxide | 2.5 |
| Sodium phosphate | 1.0 |

- 4 -

0029332

|  |  |
|---|---|
| Water | 35.0 |

Example 3.

Tooth Paste

| | |
|---|---|
| Hydroxyapatite powder | 22.3 |
| Calcium pyrophosphate | 10.0 |
| CMC sodium salt | 0.5 |
| Carrageenin | 0.6 |
| Glycerin | 20.0 |
| Sorbitol | 10.0 |
| Sodium lauryl sulphate | 2.0 |
| Flavour | 1.0 |
| Sodium saccharinate | 0.1 |
| Silicon dioxide | 2.0 |
| Sodium phosphate | 0.5 |
| Water | 30.0 |

Example 4

Tooth Paste

| | |
|---|---|
| Hydroxyapatite powder | 38.1 |
| CMC sodium salt | 1.0 |
| Carrageenin | 0.3 |
| Glycerin | 35.0 |
| Sodium lauryl sulphate | 2.0 |
| Flavour | 1.0 |
| Sodium saccharinate | 0.1 |
| Silicon dioxide | 2.5 |
| Water | 20.0 |

Example 5.

Tooth Powder

| | |
|---|---|
| Hydroxyapatite powder | 96.3 |
| Sodium lauryl sulphate | 2.0 |
| Flavour | 1.5 |
| Sodium saccharinate | 0.2 |

- 5 -

0029332

### Example 6.

Tooth Powder

| | |
|---|---|
| Hydroxyapatite Powder | 40.7 |
| Calcium pyrophosphate | 50.0 |
| Silicon dioxide | 5.0 |
| Sodium lauryl sulphate | 2.0 |
| Flavour | 2.0 |
| Sodium saccharinate | 0.3 |

### Example 7.

Wet Tooth Powder

| | |
|---|---|
| Hydroxyapatite Powder | 65.38 |
| Calcium phosphate | 10.0 |
| Sorbitol | 10.0 |
| Sodium lauryl sulphate | 2.0 |
| Flavour | 1.5 |
| Calcium phosphate | 1.0 |
| Water | 10.0 |
| Sodium saccharinate | 0.12 |

### Example 8.

A 0.1% solution of Neutral Red was applied to the front teeth of each of three male adults A,B, and C (22 to 25 years old), who had been using conventional, commercially available dentifrices. Thereafter, a similar dyeing operation was conducted one day after they started to use the dentifrice of Example 1 and the plaque-stained areas before and after the change of the dentifrice were compared. In the case of A, the stained area after the change was about 10% of the initial stained area and it was confirmed that the decontamination of the plaque areas was about 90%. In cases B and C the decontamination of the plaque areas was about 50%.

### Example 9.

Abrasive effects of dentifrices were tested using, for teeth ,plate-form bodies made of sintered hydroxyapatite having a hardness similar to that of the enamel portion of a tooth. The plate-form bodies were each subjected to brushing using tooth brushes and different dentifrices for 60 minutes. The losses in weights of the respective plate-form bodies were measured. In the result, the abrasive effects of dentifrice compositions of the present invention were similar to those of commercially available dentifrices (WHITE & WHITE and DENTER LION).

Example 10.

An adult's permanent tooth was sliced in two using a prisma adamantinum to form a section having a thickness of about 200μm. A saturated solution of hydroxyapatite was prepared by stirring hydroxyapatite powder into distilled water in quantities equivalent to 1g hydroxyapatite per 100ml water. The supernatant liquid was passed over the prepared tooth section at a flow rate of 0.6ml/min. The section was observed continuously with the use of a polarization microscope and it was confirmed that the section became coated with material resembling apatite crystals to a thickenss of about 1μm after 14 hours.

Example 11.

### Tooth Paste

| | |
|---|---|
| Hydroxyapatite powder | 10.0 |
| Calcium phosphate | 25.0 |
| CMC sodium salt | 0.3 |
| Carrageenin | 1.2 |
| Glycerin | 10.0 |
| Sorbitol | 15.0 |
| Sodium lauryl sulphate | 2.0 |
| Flavour | 1.2 |
| Sodium saccharinate | 0.1 |
| Silicon dioxide | 2.0 |
| NaCl | 0.27 |
| $MgCl_2$ | 0.01 |
| Water | 32.92 |

Example 12.

### Tooth Paste

| | |
|---|---|
| Hydroxyapatite powder | 5.0 |
| Calcium phosphate | 10.0 |
| Calcium pyrophosphate | 20.0 |
| CMC sodium salt | 1.0 |

0029332

| | |
|---|---|
| Sodium alginate | 0.1 |
| Glycerin | 10.0 |
| Sorbitol | 10.0 |
| Sodium lauryl sulphate | 1.5 |
| Sodium lauryl sarcosinate | 0.5 |
| Flavour | 0.5 |
| Sodium saccharinate | 0.1 |
| Silicon dioxide | 2.5 |
| NaCl | 3.0 |
| $MgCl_2$ | 0.2 |
| Water | 35.0 |

## Example 13

### Tooth Paste

| | |
|---|---|
| Hydroxyapatite powder | 20.0 |
| Calcium pyrophosphate | 10.0 |
| CMC sodium salt | 0.5 |
| Carrageenin | 0.6 |
| Glycerin | 20.0 |
| Sorbitol | 10.0 |
| Sodium lauryl sulphate | 2.0 |
| Flavour | 1.0 |
| Sodium saccharinate | 0.1 |
| Silicon dioxide | 2.0 |
| KCl | 2.0 |
| $MgCl_2$ | 0.3 |
| Sodium phosphate | 0.5 |
| Water | 30.0 |

## Example 14

### Tooth Paste

| | |
|---|---|
| Hydroxyapatite powder | 35.0 |
| CMC sodium salt | 1.0 |
| Carrageenin | 0.3 |
| Glycerin | 35.0 |
| Sodium lauryl sulphate | 2.0 |
| Flavour | 1.0 |
| Sodium saccharinate | 0.1 |

| | |
|---|---|
| Silicon dioxide | 2.5 |
| NaCl | 2.0 |
| $MgCl_2$ | 0.1 |
| KCl | 1.0 |
| Water | 20.0 |

Example 15

Tooth Powder

| | |
|---|---|
| Hydroxyapatite powder | 90.8 |
| Sodium lauryl sulphate | 2.0 |
| Flavour | 1.5 |
| Sodium saccharinate | 0.2 |
| NaCl | 5.0 |
| $MgCl_2$ | 0.5 |

Example 16.

Tooth Powder

| | |
|---|---|
| Hydroxyapatite powder | 38.0 |
| Calcium pyrophosphate | 50.0 |
| Silicon dioxide | 5.0 |
| Sodium lauryl sulphate | 2.0 |
| Flavour | 2.0 |
| Sodium saccharinate | 0.3 |
| NaCl | 1.8 |
| $MgCl_2$ | 0.2 |
| Potassium phosphate | 0.7 |

Example 17.

Wet Tooth Powder

| | |
|---|---|
| Hydroxyapatite powder | 63.0 |
| Calcium phosphate | 10.0 |
| Sorbitol | 10.0 |
| Sodium lauryl sulphate | 2.0 |
| Flavour | 1.5 |
| NaCl | 3.3 |
| $MgCl_2$ | 0.08 |
| Water | 10.0 |
| Sodium saccharinate | 0.12 |

Example 18

1g of hydroxyapatite powder was introduced into 100ml of distilled water at 37$^O$C and NaCl and $MgCl_2$ were added in

various concentrations. The calcium ion concentrations of the respective solutions were measured and it was confirmed that the solubility of hydroxyapatite was increased by adding $NaCl$ and $MgCl_2$. The calcium ion concentrations 10 days after the preparation of the solutions were as follows:

NaCl concentration;   0  0.3  0.3   3.0  3.0  (%)

$MgCl_2$ concentration;   0  0.03 0.3  0.03 0.3 (%)

$Ca^{++}$ concentration; 4.9 26.0 31.2 31.5 36.7 (ppm)

Example 19.

An adult's permanent tooth was sliced in two using a prisma adamantinum to prepare a section about 200μm thick. The enamel portion was removed to expose the dentine portion. A saturated solution of hydroxyapatite in distilled water containing 0.001% of $MgCl_2$ and 0.9% of NaCl was passed over the prepared tooth section at a flow rate 0.6ml/min. The section was observed continuously with the use of a polarization microscope and it was confirmed that the surfaces of the section were coated all over with material resembling apatite crystals to a thickness of about 5μm.

## CLAIMS

1.     A dentifrice composition containing synthetic hydroxyapatite powder.

2.     A dentifrice composition according to Claim 1, which is neutral or weakly alkaline.

3.     A dentifrice composition according to Claim 2, which has a pH of from 7 to substantially 9.

4.     A dentifrice composition according to Claim 1, · Claim 2 or Claim 3, in which the hydroxyapatite powder has an average particle size of about 2$\mu$ and a maximum particle size of 10$\mu$ or less.

5.     A dentifrice composition according to any of the preceding claims, further containing citric acid, lactic acid, acetic acid, pyrrolic acid, glutamine, proline, serine, glycine or a mixture thereof.

6.     A dentifrice composition according to any of the preceding claims, further containing 0.1 to 20% by weight of NaCl and/or KCl and 0.003 to 3% by weight of MgCl$_2$, based on the weight of the composition.

| Category | Citation of document with indication, where appropriate, of relevant passages | Relevant to claim |
|---|---|---|
| X | CA - A - 999 238 (KODDERMAN) <br> * Page 2, lines 1-9; page 3, line 19 - page 5, line 1; page 5, line 25 - page 6, line 3; example 1; claims 1-3,5,7 * | 1-6 |
| | FR - A - 1 434 455 (WARNER-LAMBERT) <br> * Page 1, left-hand column, lines 1-7; page 1, left-hand column, line 34 - right-hand column, line 14; page 2, left-hand column, line 14 - right-hand column, line 33; page 3, left-hand column, lines 18-57; page 4, left-hand column, line 56 - right-hand column, line 12; abstract A * <br> & GB - A - 1 090 340 | 1-3 |
| | DE - A - 295 749 (BLENDAX-WERKE) <br> * Page 1, lines 1-3; page 2, lines 12-16; claims 1,3 * | 5 |
| | FR - A - 2 255 051 (DANGELMAJER) <br> * Page 1, lines 1-3; page 2, lines 28-37 * | 5 |
| A | FR - A - 2 092 609 (DUBOIS-PREVOST) <br> * Page 1, line 39 - page 2, line 10 * | 6 |
| A | FR - A - 1 343 753 (COTTE) <br> ./. | 5 |

**DOCUMENTS CONSIDERED TO BE RELEVANT**

CLASSIFICATION OF THE APPLICATION (Int. Cl.³)

A 61 K 7/16

TECHNICAL FIELDS SEARCHED (Int. Cl.³)

A 61 K 7/16
7/20
7/22

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 16-02-1981 | CONTET |

EPO Form 1503.1  06.78

## EUROPEAN SEARCH REPORT

European Patent Office

| | DOCUMENTS CONSIDERED TO BE RELEVANT | | CLASSIFICATION OF THE APPLICATION (Int. Cl. 3) |
|---|---|---|---|
| Category | Citation of document with indication, where appropriate, of relevant passages | Relevant to claim | |
| | * Page 1, left-hand column, lines 1-17; page 1, left-hand column, lines 23-25; page 2, left-hand column, lines 26-50; page 2, right-hand column, lines 51-52 * ---- | | TECHNICAL FIELDS SEARCHED (Int. Cl. 3) |